# FASCICULE DE BREVET EUROPEEN

(11) **EP 1 189 061 B1**
(45) Date de publication et mention de la délivrance du brevet: **02.12.2009**
(21) Numéro de dépôt: 01430028.9
(22) Date de dépôt: 12.09.2001
(51) Int. Cl.: G01N 33/50, C07D 265/30, C07D 207/12, C07D 211/40

(54) **Réactif multifonctionnel pour érythrocytes mettant en jeu des carbamates et applications**
Carbamatenthaltendes Multifunktionelles Erythrozytenreagenz und Verwendungen
Multifunctional erythrocyte reagent containing carbamates and uses

(30) Priorité: 14.09.2000 FR 0011746
(43) Date de publication de la demande: 20.03.2002
(73) Titulaire: IMMUNOTECH S.A., 13276 Marseille Cedex 9 (FR)
(72) Inventeur: van Agthoven, André, 13009 Marseille (FR); Daziano, Jean-Pierre, 13013 Marseille (FR); Maples, John Allen, Miami Shores, 33138 Florida (US)
(74) Mandataire: Perrey, Ralf

(56) Documents cités:
- EP-A- 0 955 543
- WO-A-95/23873
- S. H. BOYER ET AL.: "Enrichment of erythrocytes of fetal origin from adult-fetal blood mixtures via selective hemolysis of adult blood cells: an aid to antenatal diagnosis of hemoglobinopathies." BLOOD., vol. 47, no. 6, juin 1976 (1976-06), pages 883-897, XP000196837 W.B.SAUNDERS COMPAGNY, ORLANDO, FL., US ISSN: 0006-4971
- DATABASE CHEMABS [en ligne] CHEMICAL ABSTRACTS SERVICE, COLUMBUS, OHIO, US; BACHURIN, S. O. ET AL: "Dithiocarbamates enhance the neurotoxicity of 1-methyl-4-phenyl- 1,2,3,6-tetrahydropyridine and its analogs" retrieved from STN Database accession no. 119:2666 CA XP002174120 & DOKL. AKAD. NAUK (1993), 328(3), 399-402 [BIOCHEM.], 1993,

## Description

La présente invention concerne de nouveaux réactifs et méthodes pour le traitement, et notamment la lyse des érythrocytes.

Les globules rouges étant environ mille fois plus nombreux que les leucocytes, forment une barrière pour l'analyse de la fraction leucocytaire du sang. Un traitement de sang par un réactif de lyse vide les globules rouges de leur contenu et permet de mettre ces cellules à l'écart de l'analyse. Un processus de lyse idéal comprend une lyse complète de tous les éléments érythrocytaires, sans le moindre incident sur la morphologie et la viabilité des leucocytes.

Parmi les nombreux réactifs et méthodes de lyse, on connaît les lyses hypotoniques. Mais on observe une déformation des leucocytes.

On connaît aussi les détergents, mais ceux ci produisent une détérioration de la membrane des leucocytes qui sont donc comptés par défaut.

On utilise aussi des petites molécules neutres en général lipophiles comme les alcools ou les aldéhydes mais ceux ci présentent l'inconvénient d'être toxiques pour les leucocytes.

En fait, les réactifs contenant des amines ont des performances les plus proches d'un processus de lyse idéal. Les réactifs de lyse contenant des amines comprennent le réactif au chlorure d'ammonium dont l'utilisation est très répandue et le réactif à base d'hétérocycles azotés, tel que décrit dans FR-A-2 778 413. Les réactifs de lyse au chlorure d'ammonium contiennent 155 mM NH₄Cl, 10 mM KHCO₃ et peuvent parfois aussi contenir 0,1 mM EDTA.

Néanmoins, comme décrit dans FR-A-2 778 413, le réactif de lyse au chlorure d'ammonium ayant une activité de lyse rapide et complète, a une activité toxique sur les leucocytes. Le problème de la toxicité non spécifique du chlorure d'ammonium est probablement lié à la présence d'une concentration d'ammoniaque d'environ 1 mM dans le mélange de lyse. L'utilisation de bases plus fortes, comme pyrrolidine et pipéridine avec une concentration de base libre d'environ 10 µM, résout le problème de la toxicité non spécifique, mais donne une réaction de lyse plus lente, ce qui est un inconvénient de son utilisation dans les laboratoires de routine.

EP-A-0 955 543 décrit une méthode de lyse des érythrocytes avec un hétérocycle azoté, un anticoagulant, pH 4-9 (revendications 1, 11, paragraphes 16, 19).

WO 95/23873 décrit une méthod d'enrichissement du nombre de lymphocytes foetauxdans un échantillon de sang. Il est décrit que l'échantillon de sang doit être soumis à l'action d'un inhibiteur de l'anhydrase carbonique (acétazolamide).

S.H. Boyer et al, Blood., vol 47, no 6, juin 1976, page 883-897, décrit l'intérêt d'utiliser de l'acétozolamide dans les échantillons de sang destinésau diagnostic antenatal.

Il serait donc souhaitable de disposer de nouveaux réactifs et méthodes de lyse des érythrocytes d'action plus rapide et plus complète.

Or après de longues recherches la demanderesse a découvert avec étonnement que les réactifs de lyse, et notamment les réactifs contenant des amines en présence d'un carbamate et/ou d'un catalyseur de la réaction

CO₂ + H₂O ⇄ H₂CO₃

ou/et de la réaction

carbonate ⇄ carbamate +H₂O

qui sont les deux réactions impliquées dans la formation de carbamate pendant l'absorption de CO₂ par l'érythrocyte, effectuaient une lyse des érythrocytes plus rapide et plus complète qu'en l'absence de ces réactifs.

L'influence de la présence d'un carbamate dans un réactif de lyse sur la vitesse de lyse est montrée ci-après dans la partie expérimentale.

C'est pourquoi la présente demande a pour objet un réactif multifonctionnel pour érythrocytes **caractérisé en ce qu**'il renferme une quantité suffisante pour produire la lyse ou la sphérisation des érythrocytes pour qu'ils puissent être détectés et énumérés par un cytomètre ou un appareil à numération automatique,
- d'un carbamate d'un hétérocycle azoté ou d'un halogénure tel le chlorure d'ammonium ou
- d'un agent induisant la formation par les érythrocytes, à partir de carbonate et d'un hétérocycle azoté ou d'ions ammonium, d'un carbamate associé à l'absorption de CO₂ par lesdits érythrocytes.

Le réactif multifonctionnel selon l'invention permet soit de lyser efficacement les érythrocytes soit si désirer de produire leur simple sphérisation.

Dans des conditions préférentielles de mise en oeuvre de l'invention, le carbamate peut être utilisé à la concentration molaire de 0,000001 M à 0,1M, particulièrement de 0,00001M à 0,01M et tout particulièrement de 0,0001M à 0,005M. Dans des conditions tout à fait préférentielles de mise en oeuvre du réactif de lyse décrit ci-dessus, on utilise une concentration de 0,0004M.

Dans d'autres conditions préférentielles de mise en oeuvre de l'invention, l'agent induisant la formation par les érythrocytes, à partir de carbonate ou d'une base aminée, d'un carbamate associé à l'absorption de CO₂ par lesdits érythrocytes, est un agent de catalyse de la réaction

CO₂ + H₂O ⇄ H₂CO₃

tel que l'anhydrase carbonique, par exemple l'anhydrase carbonique I ou l'anhydrase carbonique II. L'enzyme peut avoir des points isoélectriques variés et peut avoir des sources variées comme origine. L'activité de l'enzyme est exprimée en unités Wilbur-Anderson. L'anhydrase carbonique peut être présente à la concentration de 1W-A U/litre à 1.000.000 W-A U/litre, particulièrement de10W-A U/litre à 500000W-A U/litre et tout particulièrement de 100W-A U/litre à 100.000W-A U/litre. Dans les conditions tout à fait préférentielles de mise en oeuvre du réactif de lyse décrit ci-dessus, on utilise une concentration de 50.000W-A U/litre environ.

Dans la présente demande et dans ce qui suit, l'hétérocycle azoté constituant pour partie le carbamate peut être par exemple bicylique et de préférence monocyclique. Il peut être insaturé et dans ce cas comporte par exemple 5, de préférence 4, notamment 3, particulièrement 2 doubles liaisons, et il est de préférence saturé. Il comprend par exemple de 3 à 8, notamment de 3 à 6 et particulièrement de 3 à 5, et tout particulièrement 4 ou 5 atomes de carbone. Il comprend 2, notamment 1 seul atome d'azote.

On peut citer par exemple à titre d'hétérocycle azoté saturé la pyrazolidine, l'imidazolidine, l'imidazoline et la pipérazine, notamment la morpholine et particulièrement la pipéridine ou la pyrrolidine.

Dans d'autres conditions préférentielles de mise en oeuvre de l'invention, le réactif multifonctionnel pour érythrocytes renferme en outre un hétérocycle azoté ou un sel d'ammonium tel un halogénure comme du chlorure.

L'hétérocycle azoté peut être un de ceux précédemment cités.

Dans un réactif multifonctionnel selon l'invention, l'hétérocycle azoté peut être présent à la concentration molaire de 0,01 à 0,250 M, particulièrement de 0,08 à 0,19 M et tout particulièrement de 0,12 à 0,18 M. Dans des conditions tout à fait préférentielles de mise en ceuvre du réactif multifonctionnel décrit ci-dessus, on utilise une concentration de 0,17 M.

Les concentrations du composé concerné dans le milieu réactionnel (réactif multifonctionnel + échantillon sanguin) pendant la lyse érythrocytaire sont de préférence de 0,01 à 0,225 M, particulièrement de 0,072 à 0,17 M et tout particulièrement de 0,11 à 0,17 M.

Dans un réactif multifonctionnel selon l'invention utilisant l'anhydrase carbonique, on peut se dispenser de carbonate ou d'hydrogénocarbonate car le sérum sanguin en renferme naturellement. Dans encore d'autres conditions préférentielles de mise en oeuvre de l'invention, par exemple pour accélérer la lyse, le réactif multifonctionnel pour érythrocytes renferme en outre un carbonate ou un hydrogénocarbonate.

On peut citer par exemple le carbonate ou l'hydrogénocarbonate de sodium ou de potassium.

Dans un réactif multifonctionnel selon l'invention, le carbonate ou l'hydrogénocarbonate peut être présent jusqu'à la concentration molaire de 0,1 M, particulièrement jusqu'à 0,01 M, et tout particulièrement jusqu'à 0,005 M. Dans des conditions tout à fait préférentielles de mise en oeuvre du réactif multifonctionnel décrit ci-dessus, on utilise une concentration de 0,0025 M.

Les concentrations du composé concerné dans le milieu réactionnel (réactif multifonctionnel + échantillon sanguin) pendant la lyse érythrocytaire sont de préférence de 0,0001 à 0,1 M, particulièrement de 0,001 à 0,01 M et tout particulièrement de 0001 à 0,005 M.

Dans toujours d'autres conditions préférentielles de mise en oeuvre de l'invention, le réactif multifonctionnel pour érythrocytes renferme en outre un agent protecteur de la détérioration des leucocytes tel qu'un un agent de fixation, notamment un aldéhyde aliphatique tel qu'en C₁-C₅, par exemple le paraformaldéhyde et particulièrement l'aldéhyde formique.

L'aldéhyde aliphatique peut être présent dans une concentration de 0,01% à 5 %, particulièrement de 0,14% à 1% et tout particulièrement de 0,1% à 0,5%.

Dans encore d'autres conditions préférentielles de mise en oeuvre de l'invention, le réactif multifonctionnel de l'invention comprend en outre une quantité efficace d'un agent anticoagulant.

On peut citer par exemple à titre d'agent anticoagulant l'héparine, notamment l'ion citrate, l'EGTA et particulièrement l'EDTA.

Le pH du mélange sans tampon à pH neutre pendant et après la lyse a tendance à augmenter, provoquant ainsi une dégradation cellulaire. L'utilisation d'un tampon à pH environ neutre est donc souhaitable. Malgré le fait que le tampon ait tendance à inhiber la lyse, une faible quantité (par exemple 1-20 mM) de tampon peut être utilisée grâce à l'effet accélérateur d'un catalyseur tel l'anhydrase carbonique L'utilisation d'un carbamate selon l'invention permet l'utilisation d'un tampon à pH environ neutre.

C'est pourquoi dans d'autres conditions préférentielles de mise en oeuvre de l'invention, le réactif multifonctionnel de l'invention comprend en outre une quantité efficace d'un agent tampon particulièrement pH 6,5 à 7,5 . On peut citer par exemple à titre d'agent tampon le MES (Acide 2-(N-morpholino)éthane sulfonique), notamment le MOPS (Acide 3-(N-morpholino)propane sulfonique) et particulièrement l'HEPES (Acide N-(2-hydroxyéthyl)pipérazine-N'-(2-éthane sulfonique)) et particulièrement le tampon DIPSO pH 7,5.

Dans un réactif multifonctionnel selon l'invention, l'agent tampon peut être présent à la concentration molaire de 0,0001 à 0,050 M, particulièrement de 0,0005 à 0,03 M et tout particulièrement de 0,001 à 0,010 M.

Dans des conditions préférentielles de réalisation du réactif multifonctionnel ci-dessus décrit sans fixateur, on utilise 0,17 M de chlorhydrate de pyrrolidine, 2,5 mM d'hydrogénocarbonate de potassium, 3 mM de Tampon DIPSO pH 7,5, 10 mg/l d'anhydrase carbonique et 0,1 mM d'EDTA.

Dans des conditions tout à fait préférentielles de réalisation du réactif multifonctionnel ci-dessus décrit avec fixateur, on utilise en outre 0,3% d'aldéhyde formique.

Comme on l'a vu ci-dessus, l'agent de catalyse de la réaction

CO₂ + H₂O ⇄ H₂CO₃

ou

carbonate ⇄ carbamate +H₂O

tel que l'anhydrase carbonique, induit la formation par les érythrocytes, à partir de carbonate ou d'une base aminée, d'un carbamate associé à l'absorption de CO₂ par lesdits érythrocytes. On peut aussi utiliser directement les carbamates des hétérocycles azotés pour produire la lyse des érythrocytes. Certains de ceux-ci sont des produits nouveaux.

C'est pourquoi la présente demande a aussi pour objet un carbamate choisi parmi
- le carbamate de pyrrolidine,
- le carbamate de pipéridine.

Les réactifs objet de la présente invention possèdent de très intéressantes propriétés. Ils sont doués notamment de remarquables propriétés lytiques sur les érythrocytes.

Ces propriétés sont illustrées ci-après dans la partie expérimentale. Elles justifient l'utilisation des réactifs ci-dessus décrits, dans une méthode ou un procédé de lyse ou de sphérisation des érythrocytes. Une lyse des globules rouges permet d'analyser et/ou de trier à l'aide d'un cytomètre de flux les leucocytes et leurs sous populations, les plaquettes, les résidus de cellules rouges après lyse, ou tout autre élément cellulaire ou en suspension, rajouté ou non, comme par exemple des billes.

Elles justifient aussi l'utilisation des réactifs ci-dessus décrits, dans un procédé de préparation de leucocytes.

La présente demande a donc encore pour objet une méthode de lyse des érythrocytes dans laquelle on soumet à l'action d'un réactif de lyse ci-dessus décrit un échantillon de sang total traité avec un agent d'anti-coagulation comme l'EDTA, l'EGTA, l'héparine ou les ions citrate, pour produire la lyse d'au moins 95% des érythrocytes en moins de 30 minutes.

On peut opérer en l'absence ou en présence d'anticorps monoclonaux pour effectuer le marquage des cellules leucocytaires. Ces anticorps peuvent ou non être liés à un composé fluorescent tel que ceux décrits ci-après. Dans des conditions préférentielles de mise en oeuvre, ces anticorps sont liés à un composé fluorescent.

Les réactifs multifonctionnels ci-dessus décrits peuvent être utilisés comme suit :

Un échantillon de 0,1 ml de sang traité avec un agent d'anti-coagulation, et incubé préalablement avec un anticorps monoclonal ou un anticorps monoclonal conjugué à un marqueur de fluorescence, ou un mélange d'anticorps monoclonaux conjugués à des marqueurs de fluorescence est mis en contact avec 2 ml de réactif multifonctionnel ci-dessus et laissé pendant dix minutes au cours desquelles la lyse se termine. De tels marqueurs sont par exemple le CD45-FITC (CD45 conjugué à l'isothiocyanate de fluorescéine) ou le CD14 conjugué à la phycoérythrine et sont commercialisés par exemple par les sociétés DAKO, BECTON et DICKINSON ou BECKMAN COULTER. On procède alors à la lecture dans un cytomètre par exemple de type BECTON et DICKINSON Facscan ou BECKMAN COULTER XL soit immédiatement, soit jusqu'à 3 jours après la lyse.

Dans des conditions préférentielles de mise en oeuvre de la méthode de lyse selon l'invention, on choisit les conditions préférentielles ci-dessus décrites pour le réactif multifonctionnel.

Les systèmes de lyse couverts par l'invention peuvent également être utilisés en présence d'un agent de perméation comme un alcool aliphatique ou un détergent. Un réactif de perméation permettant un immunomarquage intracellulaire incluant un catalyseur tel l'anhydrase carbonique ou un carbamate fait également partie de l'invention. Une telle étape de lyse et de perméation peut être effectuée éventuellement après une fixation par un aldéhyde aliphatique.

Dans des conditions préférentielles de mise en oeuvre du procédé ci-dessus décrit, on met en contact un échantillon de sang total avec une quantité suffisante d'un réactif ci-dessus pour produire la lyse d'au moins 95% des érythrocytes en moins de 10 minutes.

Dans d'autres conditions préférentielles de mise en oeuvre du procédé ci-dessus décrit, le pH du mélange du réactif et de l'échantillon et compris entre 4 et 9.

Mais le réactif multifonctionnel selon l'invention permet aussi de produire la simple sphérisation des érythrocytes.

C'est pourquoi la présente demande a encore pour objet un procédé de sphérisation (gonflement) des érythrocytes **caractérisé en ce qu**'on met en contact un échantillon de sang total traité par un anticoagulant avec une quantité suffisante d'un réactif ci-dessus pour provoquer la sphérisation des érythrocytes de manière à permettre leur analyse par un appareil à numération automatique .

Dans des conditions préférentielles de la sphérisation des érythrocytes on met en contact un échantillon de sang avec une quantité suffisante de réactif de sphérisation pour produire une sphérisation dans un intervalle de 10 secondes à 400 secondes. Pour éviter la lyse des érythrocytes dans un intervalle de temps et pour contrôler la vitesse de la sphérisation, on peut diluer le réactif multifonctionnel selon l'invention dans une solution de préférence isotonique. On peut par exemple ajouter entre 0 et 10 fois le même volume de diluant, de préférence entre 0,5 et 5 fois, particulièrement entre 1 et 3 fois. Dans des conditions particulièrement préférées, on choisit comme solution isotonique l'IsoFlow^{®} de Beckman Coulter, qui contient NaCl : 7,92 g/l, KCI : 0,4g/l, NaH₂PO₄ : 0,19 g/l, Na₂HPO₄ : 1,95 g/l, EDTA; 0,38 g/l, 2-phénoxyéthanol : 3g/l, et NaF : 0,3 g/l.

Dans d'autres conditions préférentielles, on rajoute au réactif de sphérisation un ou plusieurs adjuvants tels que des réactifs de fixation, des colorants des acides nucléiques, et/ou des agents d'isosphérisation comme le maltoside (D.H.Tycko, US-A-5 194 909).

Comme on l'a vu ci-dessus, le réactif multifonctionnel de l'invention n'est pas agressif pour les leucocytes et peut donc être mis en oeuvre en vue de leur préparation. Les systèmes de lyse couverts par l'invention sont donc également des outils pour la préparation de cellules leucocytaires viables et peuvent remplacer la technique de séparation cellulaire sur Ficoll-Hypaque. La préparation des cellules viables après lyse de sang, sang pathologique, moelle osseuse et tout autre fluide corporel, a une application par exemple à des fins de recherche pour des essais cellulaires fonctionnels, congélation et stockage des cellules, etc. En ce qui concerne le contrôle de la viabilité cellulaire, les méthodes de lyse peuvent être utilisées avec des colorants de viabilité tels l'éosine, le bleu tryptan, le 7-AAD, le LDS 751 etc. Finalement, la lyse étant selon les études de la demanderesse, une fonction de la capacité des hématies à absorber le CO₂, le système de lyse peut être utilisé comme paramètre fonctionnel des hématies.

C'est pourquoi la présente demande a encore pour objet un procédé de préparation de leucocytes **caractérisé en ce qu'**il comprend une étape de lyse des érythrocytes utilisant un réactif multifonctionnel tel que décrit ci-dessus.

Les conditions préférentielles de mise en oeuvre des procédés ci-dessus décrits s'appliquent également aux autres objets de l'invention visés ci-dessus

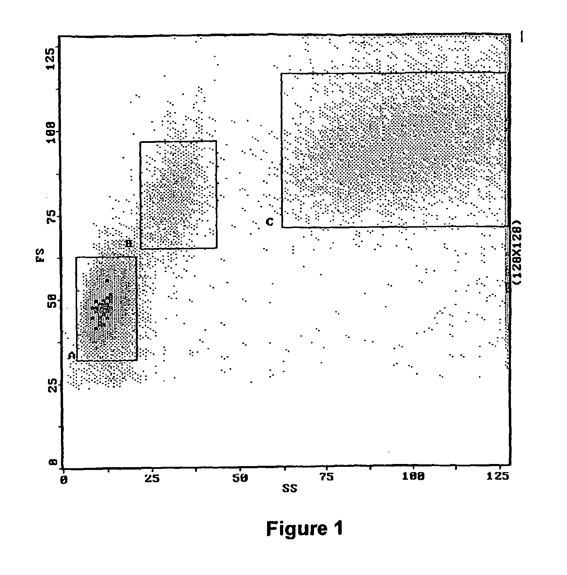

La figure 1 représente un diagramme taille-structure de diffusion d'un échantillon sanguin analysé en cytométrie en flux (BECKMAN COULTER XL) après lyse selon exemple **1**. La région A correspond aux lymphocytes, la région B correspond aux monocytes et la région C aux granulocytes.

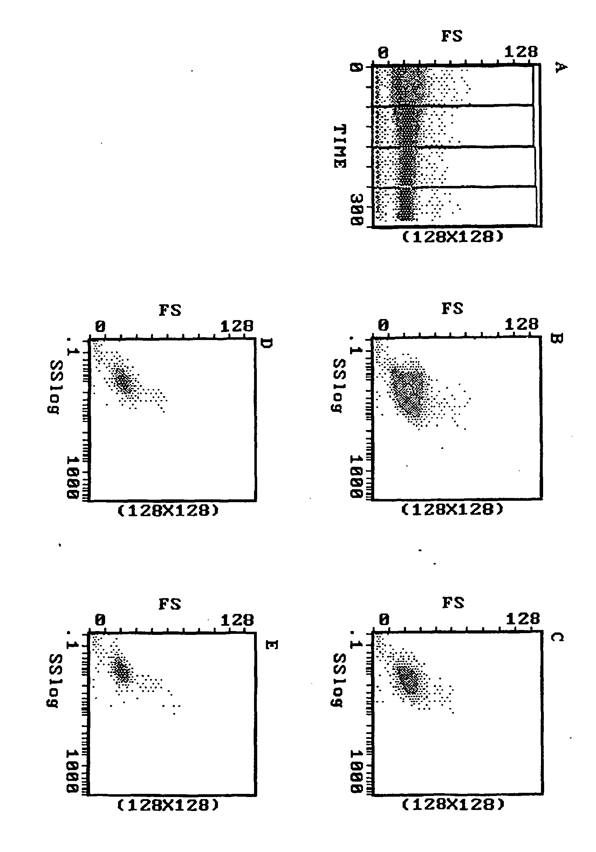

La figure 2 représente un diagramme taille-structure de diffusion d'un échantillon sanguin analysé en cytométrie en flux (BECKMAN COULTER XL),après sphérisation (sphering) selon l'exemple 6. Après contact des érythrocytes avec l'agent de sphérisation (sphering agent), décrit dans l'exemple 6, les évènements taille-structure des érythrocytes sont représentés dans 4 intervalles de 100 secondes. (figure 2A). La sphérisation et l'homogénéisation des érythrocytes qui en résulte au cours du temps sont visualisés sur les diagrammes taille-structure ; figure 2B,après 100 secondes ; figure 2C, après 200 secondes ; figure 2D, après 300 secondes ; figure 2E, après 400 secondes.

Les exemples qui suivent illustrent la présente demande.

### Exemple 1 :

On a préparé un réactif multifonctionnel pour érythrocytes ayant la composition suivante :

| | |
|---|---|
| Chlorure de pyrrolidine | 170 mM |
| Hydrogénocarbonate de potassium | 2,5 mM |
| Tampon DIPSO pH 7,5 | 3 mM |
| Anhydrase carbonique bovine (SIGMA) | 10 mg/l |
| (50000 unités W-A) | |
| EDTA | 0,1 mM |
| PH 7,3 | |

### Exemple 2 :

On a préparé un réactif multifonctionnel pour érythrocytes ayant la composition suivante :

| | |
|---|---|
| Chlorure de pyrrolidine | 170 mM |
| Hydrogénocarbonate de potassium | 2,5 mM |
| Tampon DIPSO pH 7,5 | 3 mM |
| Anhydrase carbonique bovine (SIGMA) | 10 mg/l |
| (50 000 unités W-A) | |
| EDTA | 0,1 mM |
| Formaldéhyde | 0,1 % |
| PH 7,3 | |

### Exemple 3 :

On a préparé un réactif multifonctionnel pour érythrocytes ayant la composition suivante :

| | |
|---|---|
| Chlorure de pipéridine | 170 mM |
| Hydrogénocarbonate de potassium | 2,5 mM |
| Tampon DIPSO pH 7,5 | 3 mM |
| Anhydrase carbonique bovine (SIGMA) | 10 mg/l |
| (50 000 unités W-A) | |
| EDTA | 0,1 mM |
| PH 7,3 | |

### Exemple 4 :

On a préparé un réactif multifonctionnel pour érythrocytes ayant la composition suivante :

| | |
|---|---|
| Chlorure d'ammonium | 155 mM |
| Hydrogénocarbonate de potassium | 2,5 mM |
| Tampon DIPSO pH 7,5 | 3 mM |
| Anhydrase carbonique bovine (SIGMA) | 10 mg/l |
| (50 000 unités W-A) | |
| EDTA | 0,1 mM |
| PH 7,3 | |

### Exemple 5 : Réalisation d'une lyse

On mélange 100 µl de sang total avec 1 ml d'un d'agent de lyse de l'exemple 1. On laisse reposer à température ambiante pendant 10 minutes et procède au comptage des leucocytes par cytométrie .

### Exemples 6 à 8: Préparation des carbamates de pyrrolidine, pipéridine et morpholine

Un mélange de bicarbonate de sodium et une quantité de chaque composant ( ratio molaire 1:2), a été chauffé à 100°C sous agitation. La réaction est caractérisée par une solidification du mélange, qui se produit après une heure environ. Les carbamates recherchés ont pu être récupérés après extraction par du méthanol. La structure moléculaire de ces composés synthétisés a été contrôlé par spectrométrie RMN ¹³C à 200 MHz.

Ces analyses (en méthanol deutéré) ont permis d'obtenir les déplacements chimiques (δ : ppm) suivants:
1/ Carbamate de pyrrolidine: 26,44 ; 27,24 ; 47,32 ; 47,63 (CH₂; 165,02 (C=O)
2/ Carbamate de pipéridine: 25,69 ; 26,58 ; 27,39 ; 28,71 ; 47,65 (CH₂ ; 162,76 (C=O)
3/ Carbamate de morpholine: 47,14 ; 68,42 (CH₂; 162,81 (C=O)

### Exemple 9 : Préparation d'un agent de sphérisation

Un agent de sphérisation (sphering agent) a été préparé par mélange de 2 volumes de réactif selon l'exemple 1 et un volume d'Isoflow^{®}, composition isotonique commercialisée par BECKMAN COULTER.

### Exemple 10 : Cytométrie après sphérisation

Un volume de sang de 4 microlitres a été mis en contact avec 1ml de l'agent de sphérisation de l'exemple 9. Le processus de sphérisation (sphering) a été suivi par cytométrie en fonction du temps dans un cytomètre BECKMAN COULTER XL. Les résultats sont reportés sur la figure 2 commentée ci-dessus.

### Expérimentation 1 :

On a étudié la vitesse de lyse par l'ion ammonium et par des hétérocycles azotées en présence de KHCO₃ (10 mM) ou en présence de leurs dérivés carbamates respectifs (0,4 mM). Un volume de 2 ml de réactif a été utilisé pour un volume de 0,1 ml de sang total traité par anticoagulant (EDTA). Des variations de pH ont été obtenues en rajoutant une solution aqueuse 1 M d'HCI avant l'addition de carbamate. Les carbamates (100 mM dans du méthanol) ont été ajoutés aux mélanges au début de la réaction. Le pH du mélange réactionnel a été mesuré. Le processus de lyse a été suivi en spectrophotométrie (mesure de la Densité Optique à 700 nm).Le temps de lyse est déterminé par le temps nécessaire à l'obtention d'un plateau minimum.

Les résultats obtenus sont les suivants :

| | Temps nécessaire pour arriver à une lyse totale (transparence maximale) | pH pendant la réaction de lyse |
|---|---|---|
| NH₄Cl (150 mM), KHCO₃ (10 mM) | 7 mn | 7,40 |
| Idem | 4 mn | 7,03 |
| NH₄Cl (150 mM) carbamate d'ammonium (0,4 mM) | 1 mn 50 | 7,20 |
| Pyrrolidine (150 mM), KHCO₃ (10 mM) | 7 mn | 7,20 |
| Idem | 6 mn | 7,75 |
| Pyrrolidine, carbamate de pyrrolidine (0,4 mM) | 3 mn | 7,66 |
| Pipéridine (150 mM), KHCO₃ | 15 mn | 7,30 |
| Idem | 10 mn | 7,8 |
| Pipéridine (150 mM), carbamate de pipéridine (0,4 mM) | 6 mn 40 | 7,68 |
| Morpholine (150 mM), KHCO₃ (10 mM) | 12 mn | 6,82 |
| Morpholine (150 mM), carbamate de morpholine (0,4 mM) | 8 mn | 6,90 |

Conclusion : On observe que l'addition des différents carbamates à l'ion ammonium et aux hétérocycles azotés induit une vitesse de lyse bien supérieure à celle obtenue par addition d'hydrogénocarbonate à une concentration 25 fois celle du carbamate. Comme montré dans le tableau, l'effet lytique des carbamates ne dépend pas du pH.

Expérimentation 2°. Mise en évidence du rôle catalyseur de l'anhydrase carbonique dans la réaction carbonate ⇄ carbamate +H2O;

On a étudié la réaction (NH₄)₂CO₃ ⇄ NH₂CO₂NH₄+H₂O par la conversion du carbamate d'ammonium en carbonate d'ammonium mesurée par leur différence de solubilité dans une solution aqueuse de 85% d'acétone dans laquelle le carbonate précipite et non le carbamate.

| Mélange réactionnel | Durée avant précipitation complète dans 85% d'acétone |
|---|---|
| 100 mM NH₂CO₂NH₄ | 240 min |
| 100mM NH₂CO₂NH₄ + 0,001 mg/ ml d'anhydrase carbonique | 10 min |

Conclusion°. :Les résultats montrent que l'anhydrase carbonique en plus de catalyser la conversion CO₂ + H₂O ⇄ H₂CO₃ comme bien connu, catalyse également la conversion carbonate ⇄ carbamate + H₂O

Expérimentation 3 : Mise en évidence de l'effet sur la vitesse de lyse d'anhydrases carboniques provenant de différentes sources.

Dans un réactif de lyse selon l'exemple 1, le composant anhydrase carbonique à 50.000 W-A U/l a été remplacé par des préparations d'anhydrase carbonique comme indiqué dans le tableau, chacune à 5000W-A U/l. Le processus de lyse à été suivi en spectrophotométrie (mesure de la DO à 700nm). La durée de la lyse est déterminée par le temps nécessaire à l'obtention d'un plateau minimum.

| Fournisseur | Référence | Origine | Vitesse de lyse à 5000W-A U/I |
|---|---|---|---|
| Contrôle | sans enzyme | | 15,00min |
| Sigma | C-3934 | Bovine | 7,30min |
| Biozyme | CABI | Bovine | 7,06min |
| Biozyme | CABI | Bovine | 8,30min |
| Sigma | CAII, C-2522 | Bovine | 8,51min |
| Sigma | CAII, C-6165 | Humaine | 7,06min |
| Sigma | CAI, C-4396 | Humaine | 5,06min |
| Sigma | CAI, C-5290 | Humaine | 3,24min |
| Sigma | CAI, C-1266 | Lapin | 8,00min |

Conclusion. Les différentes préparations d'anhydrase carbonique augmentent toutes la vitesse de lyse.

## Revendications

1. Utilisation d'un carbamate ou d'anhydrase carbonique, dans la préparation d'un réactif multifonctionnel pour érythrocytes destiné à produire la lyse ou la sphérisation des érythrocytes.

2. Utilisation selon la revendication 1, **caractérisée en ce que** le carbamate est un carbamate d'un hétérocycle azoté.

3. Utilisation selon l'unequelconque des revendications 1 ou 2, **caractérisée en ce que** le carbamate est choisi parmi le carbamate de pyrrolidine ou le carbamate de pipéridine.

4. Un réactif de lyse des érythrocytes contenant:
(a) une anhydrase carbonique, et
(b) un sel d'ammonium ou un hétérocycle azoté, ce dans lequel l'hétérocycle azoté est monocyclique et comprends de 3 à 6 atomes de carbone, **caractérisé en ce que** l'anhydrase carbonique est presente à la concentration de 10 à 10⁶ Wilbur-Anderson unités/litre (W-A U/litre).

5. Un réactif de lyse selon la revendication 4 ou 5, autre contenant un carbonate ou un hydrogéncarbonate.

6. Un réactif de lyse selon l'une des revendication 4 ou 5, **caractérisé en ce que** l'anhydrase carbonique est l'anhydrase carbonique I ou l'anhydrase carbonique II.

7. Un réactif de lyse selon l'une des revendication 4 à 6, **caractérisé en ce que** l'hétérocylce azoté est présent à la concentration molaire de 10 à 250 mM.

8. Un réactif de lyse selon l'une des revendication 4 à 7, **caractérisé en ce que** le sel d'ammonium est un halogénure.

9. Un réactif de lyse selon l'une des revendication 4 à 8, **caractérisé en ce que** l'hétérocycle azoté est la pyrazolidine, l'imidazolidine, l'imidazoline, la pipérazine, la morpholine, la pipéridine, ou la pyrrolidine.

10. Un réactif de lyse selon l'une des revendication 4 à 9, autre contenant un agent tampon qui est présent à la concentration molaire de 0,1 à 50 mM.

11. Un procédé pour la lyse ou pour la sphérisation des érythrocytes **caractérisé en ce qu'**on met en contact un antillon de sang total traité par un anticoagulant avec un réactif de lyse tel que défini à l'une des revendications 4 à 10.

12. Un procédé selon la revendication 11, **caractérisé en ce qu'**on met en contact un échantillon de sang total avec un réactif pour produire la lyse d'au moins 95% des érythrocytes en moins de 30 minutes.

13. Un procédé selon l'une quelconque des revendications 11 ou 12, **caractérisé en**
**ce que** le pH du mélange du réactif et de l'échantillon et compris entre 4 et 9.

14. Un procédé de préparation de leucocytes **caractérisé en ce qu'**il comprend une étape de lyse des érythrocytes utilisant un réactif de lyse tel que défini à l'une des revendications 4 à 10.

## Claims

1. Use of a carbamate or of carbonic anhydrase, in the preparation of a multifunctional erythrocyte reagent intended to produce erythrocyte lysis or spherization.

2. Use according to Claim 1, **characterized in that** the carbamate is a carbamate of a nitrogenous heterocycle.

3. Use according to either one of Claims 1 and 2, **characterized in that** the carbamate is chosen from pyrrolidine carbamate or piperidine carbamate.

4. Erythrocyte lysis reagent containing:
(a) a carbonic anhydrase, and
(b) an ammonium salt or a nitrogenous heterocycle, in which the nitrogenous heterocycle is monocyclic and contains from 3 to 6 carbon atoms, **characterized in that** the carbonic anhydrase is present at a concentration of from 10 to 10⁶ Wilbur-Anderson units/litre (W-A U/litre).

5. Lysis reagent according to Claim 4, also containing a carbonate or a hydrogen carbonate.

6. Lysis reagent according to either of Claims 4 and 5, **characterized in that** the carbonic anhydrase is carbonic anhydrase I or carbonic anhydrase II.

7. Lysis reagent according to one of Claims 4 to 6, **characterized in that** the nitrogenous heterocycle is present at a molar concentration of from 10 to 250 mM.

8. Lysis reagent according to one of Claims 4 to 7, **characterized in that** the ammonium salt is a halide.

9. Lysis reagent according to one of Claims 4 to 8, **characterized in that** the nitrogenous heterocycle is pyrazolidine, imidazolidine, imidazoline, piperazine, morpholine, piperidine or pyrrolidine.

10. Lysis reagent according to one of Claims 4 to 9, also containing a buffering agent which is present at a molar concentration of from 0.1 to 50 mM.

11. Method for erythrocyte lysis or spherization, **characterized in that** a whole blood sample treated with an anticoagulant is brought into contact with a lysis reagent as defined in one of Claims 4 to 10.

12. Method according to Claim 11, **characterized in that** a whole blood sample is brought into contact with a reagent so as to produce lysis of at least 95% of the erythrocytes in less than 30 minutes.

13. Method according to either one of Claims 11 and 12, **characterized in that** the pH of the mixture of the reagent and the sample is between 4 and 9.

14. Method for preparing leukocytes, **characterized in that** it comprises a step of erythrocyte lysis using a lysis reagent as defined in one of Claims 4 to 10.

## Patentansprüche

1. Verwendung eines Carbamats oder der Kohlenstoffanhydrase bei der Zubereitung eines multifunktionellen Reagens für Erythrozyten zur Erzeugung der Lyse oder der Sphärisierung der Erythrozyten zu erzeugen.

2. Verwendung nach Anspruch 1, **dadurch gekennzeichnet, dass** das Carbamat ein Carbamat eines Stickstoffheterozyklus ist.

3. Verwendung nach einem der Ansprüche 1 oder 2, **dadurch gekennzeichnet, dass** das Carbamat unter dem Pyrollidincarbamat oder dem Piperidincarbamat ausgewählt wird.

4. Lysereaktiv der Erythrozyten, umfassend:
(a) eine Kohlenstoffanhydrase und
(b) ein Ammoniumsalz oder ein Stickstoffheterozyklus, wobei der Stickstoffheterozyklus monozyklisch ist und 3 bis 6 Kohlenstoffatome umfasst, **dadurch gekennzeichnet, dass** die Kohlenstoffanhydrase in einer Konzentration von 10 bis 10⁶ Wilbur-Andersen-Einheiten/Liter (W-A Einh./Liter) vorhanden ist.

5. Lysereagens nach Anspruch 4, weiter umfassend ein Carbonat oder ein Wasserstoffcarbonat.

6. Lysereagens nach einem der Ansprüche 4 oder 5, **dadurch gekennzeichnet, dass** die KOhlenstoffanhydrase die Kohlenstoffanhydrase I oder die Kohlenstoffanhydrase II ist.

7. Lysereagens nach einem der Ansprüche 4 bis 6, **dadurch gekennzeichnet, dass** der Stickstoffheterozyklus in der molaren Konzentration von 10 bis 250 mM vorhanden ist.

8. Lysereagens nach einem der Ansprüche 4 bis 7, **dadurch gekennzeichnet, dass** das Ammoniumsalz ein Halogenid ist.

9. Lysereagens nach einem der Ansprüche 4 bis 8, **dadurch gekennzeichnet, dass** der Stickstoffheterozyklus das Pyrazolidin, das Imidazolidin, das Imidazolin, das Piperazin, das Morpholin, das Piperidin oder das Pyrrolidin ist.

10. Lysereagens nach einem der Ansprüche 4 bis 9, weiter umfassend ein Pfropfenmittel, das in der molaren Konzentration von 0,1 bis 50 mM vorhanden ist.

11. Verfahren zur Lyse oder zur Sphärisierung der Erythrozyten, **dadurch gekennzeichnet, dass** eine Vollblutprobe, behandelt mit einem Anticoagulans, mit einem Lysereagens, wie in einem der Ansprüche 4 bis 10 definiert, in Kontakt gebracht wird.

12. Verfahren nach Anspruch 11, **dadurch gekennzeichnet, dass** eine Vollblutprobe mit einem Reagens in Kontakt gebracht wird, um die Lyse von mindestens 95% der Erythrozyten in weniger als 30 Minuten zu erzeugen.

13. Verfahren nach einem der Ansprüche 11 oder 12, **dadurch gekennzeichnet, dass** der pH der Mischung des Reagens und der Probe zwischen 4 und 9 liegt.

14. Verfahren zur Zubereitung von Leukozyten, **dadurch gekennzeichnet, dass** es einen Schritt der Lyse der Erythrozyten unter Verwendung eines Lysereagens umfasst, wie in einem der Ansprüche 4 bis 10 definiert.
